# EUROPEAN PATENT SPECIFICATION

(11) **EP 2 853 898 B1**
(45) Date of publication and mention of the grant of the patent: **04.01.2017**
(21) Application number: 13186545.3
(22) Date of filing: 27.09.2013
(51) Int. Cl.: G01N 33/74

(54) **Analysis of myostatin in serum**
Analyse von Myostatin im Serum
Analyse de la myostatine dans le sérum

(43) Date of publication of application: 01.04.2015
(73) Proprietor: Medizinische Hochschule Hannover, 30625 Hannover (DE)
(72) Inventor: Heineke, Jörg, 30629 Hannover (DE); Breitbart, Astrid H., 30171 Hannover (DE); Scharf, Gesine M., 30823 Garbsen (DE)
(74) Representative: Taruttis, Stefan Georg

(56) References cited:
- Anonymous: "Human Myostatin ELISA - Prodomain specific", BioVendor Laboratory Medicine, Inc, 18 November 2005 (2005-11-18), XP055100354, Retrieved from the Internet: URL:http://deltaclon.es/pdf/RD193058100.pd f [retrieved on 2014-02-05]
- Anonymous: "Myostatin (Human) ELISA (EIA-4619)", , 14 November 2006 (2006-11-14), XP055100207, Retrieved from the Internet: URL:http://www.drg-international.com/ifu/e ia-4619.pdf [retrieved on 2014-02-04]
- AWANO H ET AL: "Wide ranges of serum myostatin concentrations in Duchenne muscular dystrophy patients", CLINICA CHIMICA ACTA, ELSEVIER BV, AMSTERDAM, NL, vol. 391, no. 1-2, 19 November 2007 (2007-11-19), pages 115-117, XP022607793, ISSN: 0009-8981, DOI: 10.1016/J.CCA.2008.01.024 [retrieved on 2008-02-01]
- KARL FLORIAN WINTGENS ET AL: "Plasma myostatin measured by a competitive ELISA using a highly specific antiserum", CLINICA CHIMICA ACTA, ELSEVIER BV, AMSTERDAM, NL, vol. 413, no. 15, 18 April 2012 (2012-04-18), pages 1288-1294, XP028506790, ISSN: 0009-8981, DOI: 10.1016/J.CCA.2012.04.023 [retrieved on 2012-04-25]
- LAKSHMAN K M ET AL: "Measurement of myostatin concentrations in human serum: Circulating concentrations in young and older men and effects of testosterone administration", MOLECULAR AND CELLULAR ENDOCRINOLOGY, ELSEVIER IRELAND LTD, IE, vol. 302, no. 1, 10 April 2009 (2009-04-10), pages 26-32, XP025988959, ISSN: 0303-7207, DOI: 10.1016/J.MCE.2008.12.019 [retrieved on 2009-01-21]
- Anonymous: "Myostatin Propeptide Human, Chicken Polyclonal Antibody Product Data Sheet Amino Acid Sequence", , 8 September 2008 (2008-09-08), XP055100220, Retrieved from the Internet: URL:http://search.cosmobio.co.jp/cosmo_sea rch_p/search_gate2/docs/BVL_/RD183057050.2 0081001.pdf [retrieved on 2014-02-04]
- ASTRID BREITBART ET AL: "Highly Specific Detection of Myostatin Prodomain by an Immunoradiometric Sandwich Assay in Serum of Healthy Individuals and Patients", PLOS ONE, vol. 8, no. 11, 15 November 2013 (2013-11-15), page e80454, XP055100145, ISSN: 1932-6203, DOI: 10.1371/journal.pone.0080454

## Description

The present invention relates to an analytical method for the determination of the concentration of myostatin in serum and to a kit of parts for use in the analytical method. Myostatin, a member of the TGF-β family, is produced as pre-myostatin, a disulfide-linked homodimer, which is converted by proteolytic removal of its N-terminal 24-amino acid signal peptide to promyostatin. The promyostatin is cleaved by a furin protein convertase at amino acids 240-243, generating an N-terminal fragment of 27.7 kDa, which is termed myostatin prodomain (amino acid SEQ ID NO: 1) and a C-terminal 12.4 kDa fragment, which is termed myostatin ligand (amino acid SEQ ID NO: 2) and constitutes the biolocially acitve fragment. After cleavage, two molecules of each of the myostatin ligand and of the prodomain stay non-covalently associated, forming a latent complex of approx. 80 kDa, in which the prodomain inhibits the activity of the myostatin ligand. Cleavage of the prodomain by members of the BMP1/tolloid family of metalloproteinases releases and activates the myostatin ligand. The myostatin ligand binds to the activating receptor IIB of target cells, acting as a negative regulator of muscle tissue size.

### State of art

A competitive ELISA is available from Immundiagnostik AG, 64625 Bensheim, Germany, for the determination of myostatin in human serum. The ELISA provides microtiter plates precoated with a capture antibody for myostatin. The capture antibody is incubated with a diluted sample to which labelled myostatin is added for competition. Detection of the label with a second antibody-peroxidase conjugate allows to deduce the competing concentration of myostatin contained in the original sample.

US 2013/0209489 A1 describes an antibody that binds to the mature myostatin, a 109 amino acid peptide. The antibody is shown to inhibit binding of mature myostatin to the myostatin/activin receptors of a cell line.

Hayot et al, Molecular and Cellular Endocrinology 38-47 (2011) describe the detection of myostatin from human serum in a Western blot following reducing SDS-PAGE using a rabbit polyclonal anti-myostatin antibody (Abeam) raised against the C-terminus of human myostatin.

WO 2009/058346 A1 describes antibodies which bind to epitopes on the 109 amino acid mature myostatin.

BioVendor Laboratory Medicine Inc, "Human Myostatin ELISA" (2005) describes an ELISA for the quantitative measurement of the human myostatin latent complex or free prodomain in serum using a monoclonal antibody that is labelled with biotin, followed by detection using HRP bound to streptavidin.

Wintgens et al., Clin Chim Acta 1288-1294 (2012), Immundiagnostik AG, describes the development of a competitive ELISA using a polyclonal rabbit antiscrum against recombinant human proMyo. Further, Wintgens et al. describes that the proMyo ELISA by BioVendor is not distributed.

"DRG® Myostatin (Human) ELISA, revised 14 Nov. 2006" (D2), referring to "DRG International Inc., USA, describes an ELSA corresponding to that of BioVendor.

Awano et al., Clin Chim Acta 115-117 (2008) (D3) describes measurement of myostatin in sera from Duchenne muscular dystrophy patients by the ELISA kit purchased from BioVendor (p. 115, right col., 2^{nd} paragraph). Results show a very scattered distribution of serum concentrations from below 10 to above 50 ng/dl.

Lakshman et al., Molecular and Cellular Endocrinology 26-32 (2009) (D5) describes the use of an ELISA using monoclonal antibodies directed against the ActRIIB receptor-interaction site on myostatin

Breitbart et al in Am J Physiol Heart Circ Physiol H1973-H1982 (2011) review that the mature myostatin ligand which is released from the latent complex present in serum is the active ligand which binds to the activin receptor (ActRIIB). Apart from inhibiting the mystatin ligand in the latent complex, no activity of the myostatin prodomain is reported.

### Object of the invention

It is an object of the invention to provide for an alternative analytical method suitable for detecting myostatin in serum, especially with high sensitivity, high specificity, good reproducibility, preferably in a high-throughput method. A further object is to provide a kit of parts which is adapted for use in the analytical method for detecting myostatin in serum.

### General description of the invention

The invention achieves the object by the features of the claims, especially by a process for analysis for determination of the concentration of myostatin in blood serum, in which process a first immobilized antibody specific for the myostatin prodomain is contacted with the serum and following removal of unbound components for the immobilized first antibody, a second antibody specific for the myostatin prodomain is contacted with the first antibody, followed by detection of the second antibody. By contacting the first antibody with the second antibody, the second antibody is contacted with the myostatin, which is bound to the first antibody. The first antibody and the second antibody can be different antibodies, e.g. having specificity for different epitopes of the myostatin prodomain and/or having different sections connecting the paratopic regions, e.g. having the constant chains from different animals or having synthetic sections connecting the paratopic regions, e.g. in the form of a diabody, minibody or IgG. For detection, the second antibody is labelled by coupling to a label, which can be detected directly, which is a radioactive atom. The label is then detected radiometrically. Accordingly, the method for determination of the concentration of myostatin in serum comprises the steps of contacting an immobilized first antibody having specificity for the myostatin prodomain with serum, followed by contacting the immobilized first antibody with a second antibody having specificity for the myostatin prodomain, detecting the amount of second antibody coupled to the first immobilized antibody and equating the signal detected for the second antibody bound to the first antibody to the concentration of myostatin, e.g. equating the signal to the concentration of myostatin ligand by calculating the concentration of myostatin ligand on the basis of this signal, preferably using calibration measurement of samples having known concentrations of myostatin prodomain and converting the concentration determined for the myostatin prodomain to the equimolar concentration of myostatin ligand.

In contrast to prior art methods aiming at directly detecting the concentration of the mature myostatin ligand of 109 amino acids, the analytical method of the invention is based on detecting the serum concentration of the myostatin prodomain for determination of the concentration of the myostatin ligand (SEQ ID NO: 2). Accordingly, the method determines the concentration of the myostatin ligand indirectly by detection of the myostatin prodomain, which are both contained in the latent complex. It has been found that the determination of the concentration of myostatin ligand by measuring the serum concentration of the myostatin prodomain according to the invention yields more precise results than direct measurement of the biologically active myostatin ligand, because the latter requires acid pretreatment and subsequent neutralization, in order to release the myostatin ligand from the latent complex. This step, however, often hampers subsequent detection by antibodies and it is unclear if the release of the ligand from the latent complex is complete, i.e. whether really the true amount of myostatin ligand can be quantified by the assay.

The method for determination of the serum concentration of myostatin by measuring the concentration of the latent complex using a first antibody and a second antibody, both specific for the myostatin prodomain, has the advantage of having robustness against interference by sample components. For example, it was determined that the addition of possible interfering sample components selected from lipid particles up to 5 mM, bilirubin up to 300 µM, albumin up to 40g/l, hemoglobin up to 5 g/l or of urea up to 2 g/l gave the same deviation of the determined concentration from a pre-set myostatin prodomain concentration of <20% as found for the same samples without addition of possible interfering components. This was found for normal levels and for high levels of myostatin in serum samples. Further, it was determined that the addition of the mature myostatin ligand to serum samples did not influence the result of the analytical method, also confirming the specificity of the method to detect the concentration of the latent complex by binding its myostatin prodomain portion. For determination of the concentration of myostatin ligand, the molar concentration of myostatin prodomain measured by binding to the first and second antibodies with subsequent detection of the second antibody can be calculated and equated to the molar concentration of myostatin ligand. For calculating, 1ng myostatin prodomain equals 36.09 fmol. 36.09 fmol myostatin ligand equals 0.44778 ng myostatin ligand. Correspondingly, the concentration in ng/ml myostatin prodomain multiplied by a factor of 0.44778 gives the concentration in ng/ml myostatin ligand.

Further, it was found that the result obtained by the method is not influenced to a significant extent by freezing serum samples prior to the analysis, and the result is not influenced to a significant extent by exposing the samples to room temperature prior to analysis, e.g. for 24 or 48 h. In detail, the measurement using radioactive quantification showed no significant loss of signal for samples that were stored at room temperature for 48 h (105.9-111.7%) compared to samples immediately frozen (100% with immediate freezing). Further, four cycles of freezing and thawing of samples containing lower than normal, normal or higher than normal concentrations of added myostatin prodomain confirmed that the result obtained by the method is not influenced to a significant extent by freezing serum samples prior to the analysis (87.4-118.4% compared to initial 100%). Accordingly, the method can comprise the step of freezing the serum sample, or storing the serum sample maximally at room temperature, e.g. at 20°C for up to 48 h.

Another advantage of the method according to the invention is the high specificity for the detection of myostatin latent complex in serum, which was verified independently by size-exclusion chromatography (SEC), myostatin-ligand activity (CAGA-luciferase) assay as well as detection of myostatin prodomain and ligand in Western-blotting of the fractions from the SEC, for which the method determined high concentrations of myostatin ligand.

Generally, the serum can be prepared from whole blood by the steps of centrifugation for 10 minutes with 1000 x g and subsequent decantation of the serum.

The first and second antibodies are polyclonal. Preferably, the first antibody and the second antibody have no cross-reactivity with the myostatin ligand.

When analysing sera from 169 stable chronic heart failure patients without cachexia, no significant increase of the myostatin concentration was found in comparison to the concentrations determined in sera from 249 healthy persons. Accordingly, the serum preferably originates from chronic heart failure patients with cachexia. When analysing sera of 53 patients with gastrointestinal or hepatic cancer with strong recent weight loss, no increase in serum myostatin levels was detected. Therefore preferably, sera from patients with other tumors (e.g. lung carcinoma, renal carcinoma) that induce myostatin dependent cachexia are analyzed by the method. Further preferably, the serum originates from a pulmonary disease patient, especially from an underweight pulmonary disease patient, as it has been found that in underweight pulmonary disease patients, a profound rise in the concentration of myostatin was determined by the analytical method of the invention. Sera of patients with cachexia due to HIV infection or chronic renal disease can also be preferably analysed in the method of this invention.

As the method of the invention does not require the separation of the myostatin ligand from its latent complex with the myostatin prodomain, the method is preferably free from a treatment step for separating the myostatin latent complex for separating the myostatin ligand from the myostatin prodomain, e.g. the method preferably is free from proteolytic treatment or acid treatment of the serum prior to contacting with the first and second antibodies. Accordingly, the method preferably is free from a step of treatment of the serum separating the myostatin ligand from the myostatin prodomain. Preferably, in the method the serum is obtained by separating cells from whole blood, e.g. by centrifugation, and separating the heavier fraction from the serum, optionally with freezing and thawing of the serum prior to contacting with the antibodies.

Preferably, for calibration, the method comprises the parallel treatment of at least one sample having a known concentration of myostatin prodomain, and to convert the signal measured from the second antibody bound to the immobilized first antibody by applying the relation of the signal intensity measured for the sample of known myostatin prodomain concentration on the signal measured for the serum sample. For example, the method in addition to treating the serum, comprises the treatment of at least one sample containing a known concentration of myostatin prodomain separate from the serum sample and by the same method steps.

Accordingly, the kit of parts preferably contains samples having a known concentration of myostatin prodomain for use as a calibrating sample to be run in parallel to the serum in the method, each by the same steps of the method.

### Detailed description of the invention

The invention is now described in greater detail by way of examples with reference to the figures, showing in
- Fig. 1 the measurement results of the analytical method of the invention obtained for serum fractions of two sera from size-exclusion chromatography (SEC),
- Fig. 2 the measurement results from a myostatin ligand reporter assay for SEC serum fractions,
- Fig. 3 a Western blot of SEC serum fractions with detection using an anti-myostatin propeptide antibody,
- Fig. 4 a Western blot of SEC serum fractions with detection using an anti-myostatin ligand antibody,
- Fig. 5 measurement results of the analytical method of the invention for age groups of apparently healthy persons,
- Fig. 6 measurement results of the analytical method of the invention for apparently healthy female and male persons aged at least 55 years,
- Fig. 7 measurement results of the analytical method of the invention for healthy persons and different patient groups,
- Fig. 8 shows the measurement results of the analytical method of the invention obtained for serum fractions from chronic pulmonary disease patients, and
- Fig. 9 comparative measurement results obtained for the SEC serum fractions by a commercial myostatin ELISA.

### Example 1: Analysis of sera and of SEC serum fractions

Two serum samples obtained from heart failure patients were analysed by the analytical method of the invention. The first antibody was raised against the 243 amino acid recombinant human myostatin propeptide, i.e. lacking the signalling sequence, with N-terminally added amino acids MG and C-terminally added amino acids KLN. The antibody was obtained from BioVendor GmbH, Heidelberg, Germany under catalogue No. RD183057050. The later analysis using SEC and Western blotting revealed that this antibody is specific for the myostatin prodomain.

For the analytical method, the anti-myostatin prodomain antibody, subsequently also referred to as anti-MP, was immobilized to the surface of tubes (Nunc Maxisorp) serving as carrier. For immobilisation, the carrier was contacted with 0.5 µg anti-MP in 0.2 ml sodium carbonate buffer (0.1M, pH 9.5) overnight, then washed three times using PBS (40mM sodium hydrogen phosphate, 150 mM NaCl, 0.1% Tween20, pH 7.4) and blocked with casein buffer (10 g/l casein, 5 g/l bovine serum albumin (BSA), 0.1 M sodium carbonate buffer, pH 9.5) for 1 h at room temperature. Following two washing steps, the immobilized anti-MP was contacted with 100 gl serum in admixture with 100 µl assay buffer (40 mM sodium hydrogen phosphate, 0.1% sodium azide, 5 g/l BSA, 10 ml/l mice serum, pH 7.4) and incubated over night at 4°C. The second antibody for detection of myostatin prodomain bound to the first antibody was the same anti-MP labelled with ¹²⁵I (5 MBq) using the chloramine-T method according to Seevers et al (Chemical Reviews 82: 575-590 (1982)) and purified by SEC on a Sephadex G-25 column. Samples and sample buffer were removed from the carrier and the carrier was washed three times before the second antibody was added (100 µg/ml in assay buffer). Incubation was overnight at 4°C, and after three washing steps, detection was in a gamma-counter (LKB Wallac 1261). For calibration, sample buffer containing different concentrations from 0 to 100 ng/ml of myostatin prodomain (obtained from Prospec, cat. No. cyt-448-b) were analysed in parallel. Using the signals detected for the calibration samples, the concentrations of myostatin ligand were determined as the measured concentrations of myostatin prodomain. For one sample, a concentration of 1629 ng/ml myostatin prodomain was measured, from which 729.4 ng/ml myostatin ligand were determined for the other sample, a concentration of 1.37 ng/ml myostatin prodomain was measured, from which 0.61 ng/ml myostatin ligand was determined.

Using serial dilutions of 6 different serum samples having normal concentrations of myostatin as determined by the analytical method of the invention, it was found that the method showed linearity for dilutions with deviations between measured and calculated concentrations of <25% in each sample for concentrations ranging from 0.89556 to 217.17 ng/ml myostatin ligand.

Using samples of 1.25 to 100 ng/ml myostatin prodomain for calibration, the detection limit was found to 0.5 ng/ml myostatin prodomain, from which a detection limit of 0.22 ng/ml myostatin ligand is determined, determined as mean plus 3 standard deviations in 10 repetitive measurements of the 0 sample. The limit of quantification was 1.25 ng/ml, corresponding to 0.56 ng/ml myostatin ligand, determined as the lowest concentration with an imprecision of <20% in repetitive measurements. The imprecision within one set of analyses according to the method (within one assay of several samples) ranged from 3.7 to 14.1% as determined in 10 parallel duplicate measurements of 11 serum samples containing 1.5 to 100.4 ng/ml myostatin prodomain, corresponding to 0.67 to 44.96 ng/ml myostatin ligand. Imprecision between sets of analyses according to the method (between assays) ranged from 10.0 to 18.9% as determined in 13 separate assays of 7 pooled serum samples containing 7.4 to 58 ng/ml myostatin prodomain, corresponding to 3.3 to 26 ng/ml myostatin ligand, on different days by two different technicians and with different lots of first and second antibodies and myostatin prodomain calibration samples and different tubes.

An aliquot of 1 ml of the serum, measured to contain 1629 ng/ml myostatin prodomain and therefrom determined to contain 729.4 ng/ml myostatin ligand as described above was used as a high myostatin sample, and an aliquot of 1 ml of the serum measured to contain 1.37 ng/ml myostatin prodomain and therefrom determined to contain 0.61 ng/ml myostatin ligand as described above was used as a low myostatin sample. Each sample was fractionated by SEC on a Sephadex G-100 column to 75 fractions. Column equilibration was by TRIS (20 mM TRIS, 150 mM NaCl, 1 g/l sodium azide, 5g/l BSA, pH 7.0). Dextran blue (2000 kDa, Pharmacia) was used to determine the column void volume, mass standards were BSA (66 kDa), recombinant myostatin propeptide (27.8 kDa) and insulin (5.8 kDa, Lilly). For each fraction, the concentration of the myostatin ligand was determined according to the analytical method of the invention as described above using the anti-MP as capture first antibody and ¹²⁵I-labelled anti-MP as second antibody with radiometric detection.

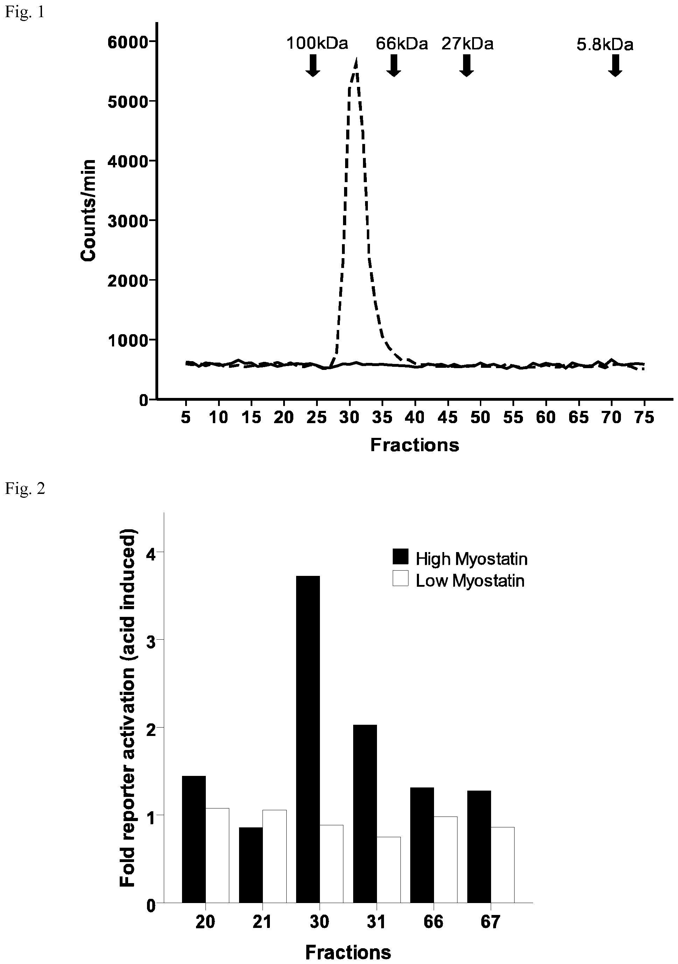

The results are shown in Fig. 1, wherein the dashed line gives represents the high myostatin sample and the drawn line represents the low myostatin sample. The mass standards are indicated by arrows. It can be seen that only the high myostatin sample gives a detection peak, which is at fractions 30 to 31, whereas no detection peak was measured for the low myostatin sample. The size of the protein eluted at the detection peak as deduced from the mass standards is 66-100 kDa, which corresponds to the size of the latent complex.

The biological activity of the protein of the peak SEC fractions was analysed in a SMAD (CAGA) luciferase reporter assay. HEK293 cells (obtained from ATCC) were transfected with pGL3(CAGA)₁₂ reporter plasmid encoding an expression cassette for luciferase, the transcription of which is dependent on activation of the SMAD protein by the myostatin ligand (according to Zhu X. et al., Cytokine, 26 (2004) 262-272). Cells were plated on 48 well plates. For separation of the myostatin ligand from the latent complex, 100 µl from an SEC fraction was incubated with 50 µl 1N HCl for 10 min and neutralized by adding 50 µl 1.2N NaOH, 0.5 M HEPES. 50 µl of the acid-treated SEC fraction, or 25 µl from the SEC fractions indicated in Fig. 2 were added to wells. Cultivation was for 24 h under cell culture conditions, then cells were lysed and luciferase activity was measured according to standard procedures. Measurement results were normalized for total protein content per well as determined by a total protein assay (Micro BCA protein assay kit, Thermo Scientific). Fig. 2 shows the activation factor (fold activation) of specific luciferase activity (per µg total protein) obtained by acid treatment for the SEC fractions indicated for the high myostatin sample (High Myostatin) and for the low myostatin sample (Low Myostatin). Accordingly, a factor of 1 corresponds to no activation, i.e. no release of myostatin ligand from latent complex. Acid treatment led to biologic activity triggered by myostatin only for the high myostatin peak fractions, whereas the corresponding low myostatin fractions essentially show no significant myostatin activity.

This luciferase reporter assay shows that only the peak fraction identified by the analytical method according to the invention contained myostatin ligand that was released from the latent complex by the acid treatment.

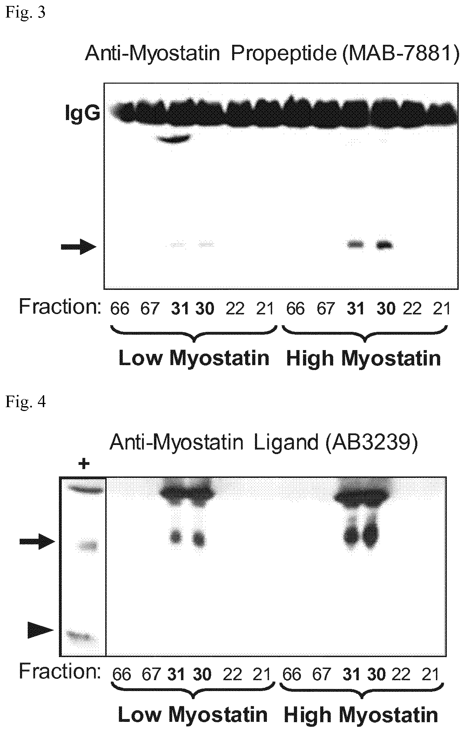

For further confirmation that the analytical method of the invention is specific for the latent complex, Western blots were made from aliquots of the SEC fractions. From the SEC fractions indicated in Figures 3 and 4, equal amounts of total protein were separated by SDS-polyacrylamide gel electrophoresis (SDS-PAGE), followed by transfer of the separated proteins onto a membrane. For detection, a monoclonal anti-myostatin propeptide antibody (Catalog No. MAB7881, R&D Systems) was used in Fig. 3, and a polyclonal anti-myostatin antibody (Catalog No. AB3239, Millipore) was used for detection of the myostatin ligand in Fig. 4. In Figs. 3 and 4, the origin of the fractions from the high myostatin sample (High Myostatin) and from the low myostatin sample (Low Myostatin) is indicated.

In Fig. 3, the arrow indicates 25 kDa, showing that the peak signal fractions 30, 31 from the high myostatin serum sample only contain significant concentrations of myostatin prodomain.

In Fig. 4, the lane + denotes recombinant myostatin ligand as a positive control, and the arrow indicates 25 kDa, as the myostatin ligand (12.5 kDa) mainly appears as a dimer on the blot. The Western blot shows that the peak signal fractions 30, 31 from the high myostatin sample contain a significantly higher concentration of myostatin ligand. The SEC fractions without detected signal by the analytical method also did not contain any myostatin in Western blotting.

These Western blot analyses show that the analytical method of the invention specifically detected the latent complex, which contained both the myostatin prodomain and the myostatin ligand.

### Example 2: Analysis of mystatin in serum samples of healthy persons and in patients

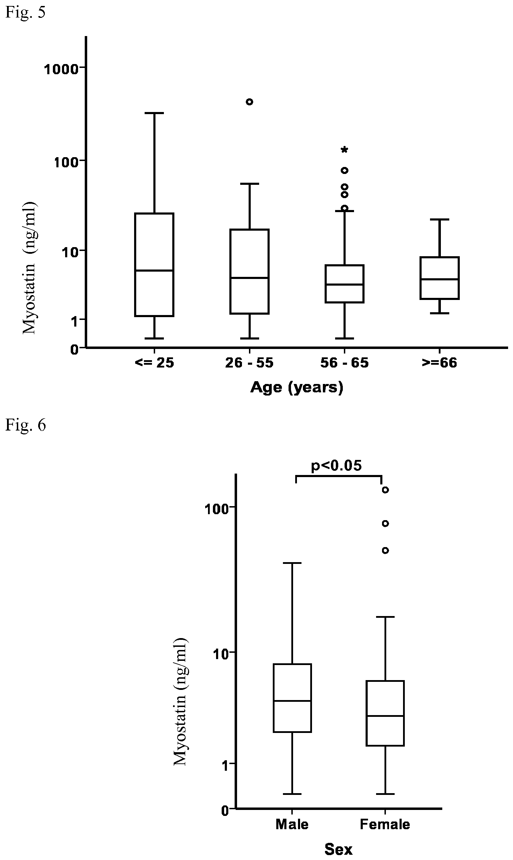

The analytical method was made according to Example 1 using serum samples from 249 apparently healthy blood donors. The results determined for the mystatin ligand on the basis of the measurement of the latent complexusing a first and a second antibody, both specific for the myostatin prodomain (also termed IRMA herein), showed no statistically significant differences. In detail, the mystatin ligand concentration was determined to 2.7 ng/ml for persons (n=63) younger than 56 years (measured as 6.0 ng/ml myostatin prodomain, 25^{th}-75^{th} percentiles 1-6 ng/ml) and in persons n=186) older than 55 years, the myostatin ligand concentration was determined to 1.7 ng/ml (measured as 3.9 ng/ml myostatin prodomain, 25^{th}-75^{th} percentiles 2-7 ng/ml). Results are shown in Fig. 5, wherein mystatin measurement results are given as ng/ml myostatin prodomain.

When comparing myostatin ligand concentrations in sera from the group over 55 years between women and men, it was found that women had a significantly lower concentration than men. Results are shown in Fig. 6, wherein mystatin measurement results are given as ng/ml myostatin prodomain.

The IRMA analysis was done using sera from patients (n=169) with chronic heart failure (CHF), of which 117 (69.2%) were in New York Heart Association (NYHA) class II and 52 (30.8%) in class III. These patients did not suffer from underweight, nor did they report current weight loss. Echocardiography data indicated severely impaired ejection fraction (EF) in 109 (64.5%) patients, moderately impaired EF in 29 (17.2%), slightly decreased EF in 7 (4.1 %) patients and in 6 patients (3.6%) no EF. Myostatin ligand was determined by IRMA in 143 (84.6%) of these patients. The median concentration was determined to 1.16 ng/ml myostatin ligand (measured as 2.6 ng/mL myostatin prodomain, 25^{th} to 75^{th} percentiles 1-6 ng/ml), which was significantly lower than the concentration found in the older healthy persons (p<0.001). As expected for CHF patients, serum concentrations of NT-proBNP and GDF15 were elevated. No significant correlation was found to age, BMI, creatinine, GDF15, NT-proBNP, systolic blood pressure or NYHA classification. The myostatin concentration was positively correlated to serum CRP (C-reactive protein) (rho=0.156, p=0.043).

IRMA analysis was done using sera from gastrointestinal cancer (GI-Ca) patients undergoing acute weight loss. Myostatin was determined in 28 of 53 patients, but no myostatin was found in 25 patients. The myostatin concentrations were significantly lower (median determined to 0.49 ng/ml myostatin ligand, measured as 1.1 ng/ml myostatin prodomain with 25^{th} to 75^{th} percentiles 0.3 ng/ml to 7.7 ng/ml myostatin prodomain; p<0.001) compared to the older healthy group. The GI-Ca patients despite the weight loss still had a normal BMI (median 23.4 kg/m²). No correlation of myostatin ligand concentrations with BMI, age, weight loss or CRP were found.

IRMA analysis was done using sera from chronic pulmonary disease (CPD) patients. In 4 of 44 patients, no myostatin ligand concentration was found. In the CPD patients group, the myostatin ligand concentration was significantly elevated, median 12.05 ng/ml median (measured as median 26.9 ng/ml myostatin prodomain; 25^{th} to 75^{th} percentiles 5 to 100ng/ml; p<0.001) compared to the corresponding healthy age group under 55 years. These patients had a very low BMI (median 17.5 kg/m²; 25^{th} to 75^{th} percentiles 16.9 to 18.3 kg/m²; p<0.001) but did not show weight loss over the last 12 months. No correlation of the mystatin concentration with age, weight loss, BMI or CRP was found for these patients.

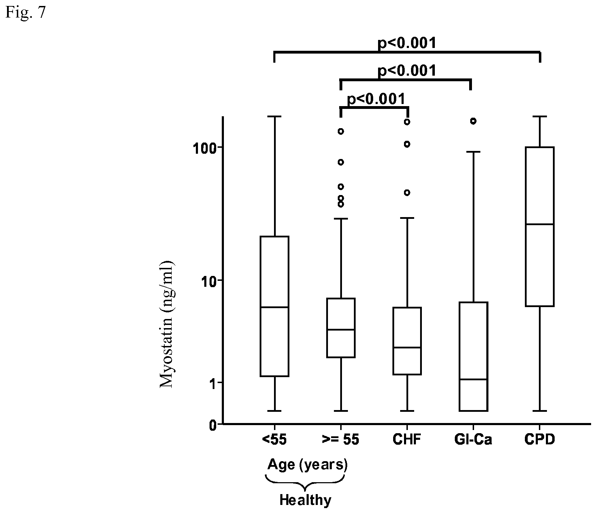

Fig. 7 shows measurement results for the above patient groups, wherein mystatin measurement results are given as ng/ml myostatin prodomain.

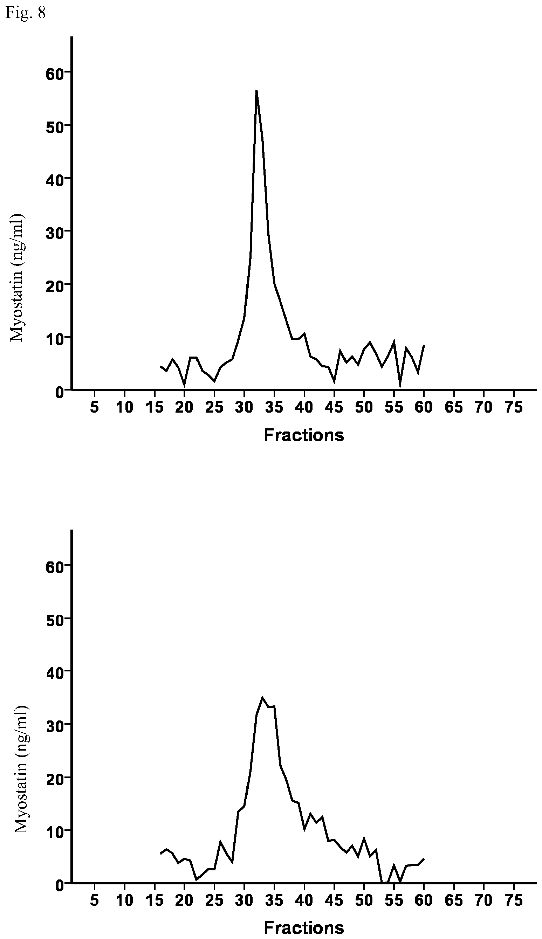

The SEC fractionation described in Example 1 was repeated for serum from chronic pulmonary disease patients, and fractions were analysed by the IRMA according to the invention. The results of mystatin ligand concentrations for each fraction are depicted in Fig. 8, showing that the main mystatin ligand concentration was found in fraction 31. These results support that the method for determination of the mystatin ligand concentration by IRMA is specific for the mystatin ligand, especially in serum of chronic pulmonary disease patients.

### Comparative example: Analysis of SEC serum fractions using a commercial ELISA

Using the SEC fractions from the high myostatin serum of Example 1, analysis of myostatin was made using the ELISA available from Immundiagnostik AG according to the manufacturer's instructions. The result is shown in Fig. 8 for the SEC fractions indicated. Arrows indicate the mass standards from SEC. A comparison of the myostatin concentrations determined for the SEC fractions with the mass standards shows that no correlation between size and concentration as determined was found. Further, this ELISA found myostatin in all of the fractions assayed, which indicates lack of specificity.

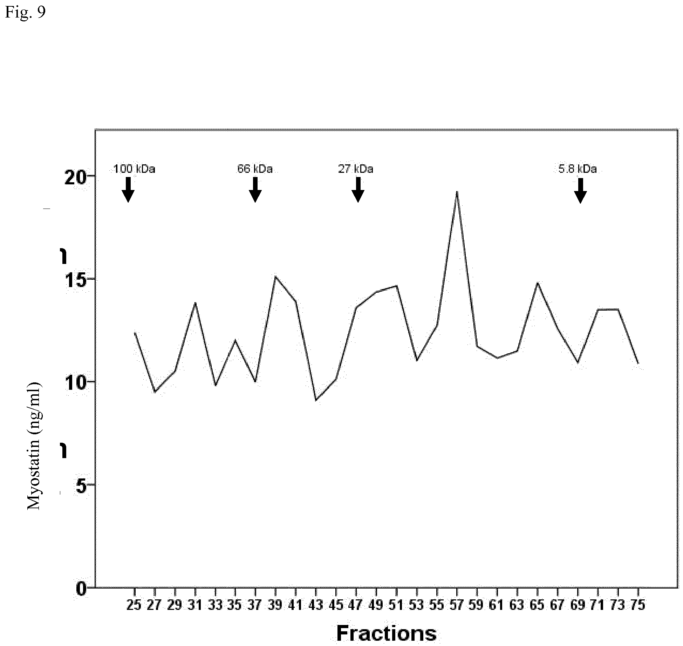

A comparison of the results of Fig. 9 with the results shown in Fig.1 for the analytical method of the invention shows the drastically improved specificity of the method of the invention.

## Claims

1. Method for determination of the concentration of myostatin in serum, the method comprising the steps of contacting an immobilized first antibody having specificity for the myostatin prodomain with serum, followed by contacting the immobilized first antibody with a second antibody having specificity for the myostatin prodomain, detecting the amount of second antibody coupled to the first immobilized antibody and equating the signal detected for the second antibody bound to the first antibody to the concentration of myostatin, **characterized in that** the second antibody is labelled with a radioactive atom and detecting the second antibody is radiometrically and **in that** the first and second antibodies are polyclonal.

2. Method according to claim 1, **characterized in that** the serum is serum from a patient having or being suspected of having a pulmonary disease or serum from a chronic heart failure patient, a HIV patient, a chronic renal disease patient or a cancer patient.

3. Method according to one of the preceding claims, **characterized in that** the first antibody and the second antibody are generated from an animal or a cell from an animal originally immunized with purified protein having or consisting of the amino acid sequence of the myostatin prodomain which is free from epitopes of the myostatin ligand.

4. Method according to one of the preceding claims, **characterized in that** the first antibody and the second antibody each independently have no specificity for the myostatin ligand.

5. Method according to one of the preceding claims, **characterized in that** the first antibody, except for being immobilized, is identical to the second antibody, which in addition is labelled.

6. Method according to one of the preceding claims, **characterized by** not comprising a step of treatment of the serum suitable for separating the myostatin ligand from the myostatin prodomain.

7. Method according to one of the preceding claims, **characterized in that** the myostatin prodomain has the amino acid SEQ ID NO: 1 and myostatin is the myostatin ligand having amino acid sequence of SEQ ID NO: 2.

8. Method according to one of the preceding claims, **characterized in that** in addition to treating the serum, at least one sample containing a known concentration of myostatin prodomain is treated separately by the same steps.

9. Method according to one of the preceding claims, **characterized by** freezing the serum prior to the analysis or by storing the serum maximally at 20 °C for up to 48 h.

10. Use of a kit of parts in a method according to one of the preceding claims, comprising a first polyclonal antibody having specificity for the myostatin prodomain, wherein the first antibody is provided for immobilization to a carrier, and a second polyclonal antibody having specificity for the myostatin prodomain, which second antibody is labelled with a radioactive atom.

11. Use of a kit of parts according to claim 10, **characterized in that** the first antibody is immobilized to a carrier.

12. Use of a kit of parts according to one of claims 10 to 11, **characterized in that** the myostatin prodomain has the amino acid SEQ ID NO: 1 and myostatin is the myostatin ligand having amino acid sequence of SEQ ID NO: 2.

13. Use of a kit of parts according to one of claims 10 to 12, **characterized in that** the first antibody, except for being immobilized, is identical to the second antibody, which in addition is labelled.

## Patentansprüche

1. Verfahren zur Bestimmung der Konzentration von Myostatin in Serum, wobei das Verfahren die Schritte des Kontaktierens eines immobilisierten ersten Antikörpers mit Serum umfasst, der eine Spezifität für die Myostatin-Prodomäne, gefolgt vom Kontaktieren des immobilisierten ersten Antikörpers mit einem zweiten Antikörper, der eine Spezifität für die Myostatin-Prodomäne hat, Detektieren der Menge des zweiten Antikörpers, die an den ersten immobilisierten Antikörper gekoppelt ist, und Gleichsetzen des für den zweiten Antikörper detektierten Signals, der an den ersten Antikörper gebunden ist, zur Konzentration von Myostatin, **dadurch gekennzeichnet, dass** der zweite Antikörper mit einem radioaktiven Atom markiert ist und das Detektieren des zweiten Antikörpers radiometrisch ist und dadurch, dass die ersten und zweiten Antikörper polyklonal sind.

2. Verfahren nach Anspruch 1, **dadurch gekennzeichnet, dass** das Serum von einem Patienten ist, der eine Lungenkrankheit hat oder bei dem der Verdacht auf eine Lungenkrankheit besteht oder Serum von einem Patienten mit chronischer Herzinsuffizienz, einem HIV-Patienten, einem Patienten mit chronischer Nierenkrankheit oder einem Krebspatienten.

3. Verfahren nach einem der voranstehenden Ansprüche, **dadurch gekennzeichnet, dass** der erste Antikörper und der zweite Antikörper aus einem Tier oder einer Zelle eines Tiers erzeugt wurden, das ursprünglich mit gereinigten Protein geimpft wurde, das die Aminosäuresequenz der Myostatin Prodomain, die frei von Epitopen des Myostatin-Liganden ist, aufweist oder daraus besteht.

4. Verfahren nach einem der voranstehenden Ansprüche, **dadurch gekennzeichnet, dass** der erste Antikörper und der zweite Antikörper jeweils unabhängig voneinander keine Spezifität für den Myostatin-Liganden haben.

5. Verfahren nach einem der voranstehenden Ansprüche, **dadurch gekennzeichnet, dass** der erste Antikörper, außer dass er immobilisiert ist, identisch zum zweiten Antikörper ist, der zusätzlich markiert ist.

6. Verfahren nach einem der voranstehenden Ansprüche, **dadurch gekennzeichnet, dass** es keinen Schritt der Behandlung des Serums umfasst, der für das Trennen des Myostatin-Liganden von der Myostatin-Prodomäne geeignet ist.

7. Verfahren nach einem der voranstehenden Ansprüche, **dadurch gekennzeichnet, dass** die Myostatin-Prodomäne die Aminosäuresequenz SEQ ID NO: 1 hat und Myostation der Myostation-Ligand ist, der die Aminosäuresequenz SEQ ID NO: 2 hat.

8. Verfahren nach einem der voranstehenden Ansprüche, **dadurch gekennzeichnet, dass** zusätzlich zur Behandlung des Serums wenigstens eine Probe, die eine bekannte Konzentration der Myostatin-Prodomäne enthält, getrennt durch dieselben Schritte behandelt wird.

9. Verfahren nach einem der voranstehenden Ansprüche, **gekennzeichnet durch** das Gefrieren des Serums vor der Analyse oder **durch** Lagern des Serums bei max. 20°C für bis zu 48 h.

10. Verwendung eines Satzes von Teilen in einem Verfahren nach einem der voranstehenden Ansprüche, umfassend einen ersten polyklonalen Antikörper, der Spezifität für die Myostatin-Prodomäne hat, wobei der erste Antikörper zur Immobilisierung an einem Träger bereitgestellt ist, und einen zweiten polyklonalen Antikörper, der Spezifität für die Myostatin-Prodomäne hat, wobei der zweite Antikörper mit einem radioaktiven Atom markiert ist.

11. Verwendung eines Satzes von Teilen nach Anspruch 10, **dadurch gekennzeichnet, dass** der erste Antikörper an einem Träger immobilisiert ist.

12. Verwendung eines Satzes von Teilen nach einem der Ansprüche 10 bis 11, **dadurch gekennzeichnet**, das die Myostatin-Prodomäne die Aminosäuresequenz SEQ ID NO: 1 hat und Myostatin der Myostatin-Ligand ist, der die Aminosäuresequenz von SEQ ID NO: 2 hat.

13. Verwendung eines Satzes von Teilen nach einem der Ansprüche 10 bis 12, **dadurch gekennzeichnet, dass** der erste Antikörper, außer dass er immobilisiert ist, identisch mit dem zweiten Antikörper ist, der zusätzlich markiert ist.

## Revendications

1. Procédé de détermination de la concentration de la myostatine dans le sérum, le procédé comprenant les étapes comprenant mettre en contact un premier anticorps immobilisé ayant une spécificité pour le prodomaine de myostatine avec du sérum, suivi de la mise en contact du premier anticorps immobilisé avec un second anticorps ayant une spécificité pour le prodomaine de myostatine, de détection de la quantité de deuxième anticorps couplé au premier anticorps immobilisé et l'assimilation du signal détecté pour le deuxième anticorps lié au premier anticorps à la concentration de la myostatine, **caractérisé en ce que** le deuxième anticorps est marqué par un atome radioactif et la détection du deuxième anticorps est radiométrique et **en ce que** le premier anticorps et le deuxième anticorps sont des anticorps polyclonaux.

2. Procédé selon la revendication 1, **caractérisé en ce que** le sérum est un sérum d'un patient ayant ou suspecté d'avoir contracté une maladie pulmonaire ou du sérum à partir d'un patient souffrant d'une insuffisance cardiaque chronique, d'un patient atteint du VIH, d'un patient souffrant d'une maladie rénale chronique ou d'un patient atteint d'un cancer.

3. Procédé selon l'une quelconque des revendications précédentes, **caractérisé en ce que** le premier anticorps et le deuxième anticorps sont générés à partir d'un animal ou une cellule provenant d'un animal immunisé initialement avec de la protéine purifiée possédant, ou consistant en la séquence d'acide aminé du prodomaine de myostatine qui est exempte d'épitopes du ligand de la myostatine.

4. Procédé selon l'une quelconque des revendications précédentes, **caractérisé en ce que** le premier anticorps et le deuxième anticorps ont chacun indépendamment aucune spécificité pour le ligand de la myostatine.

5. Procédé selon l'une quelconque des revendications précédentes, **caractérisé en ce que** le premier anticorps, à ceci près qu'il est immobilisé, est identique au deuxième anticorps, qui de plus est marqué.

6. Procédé selon l'une quelconque des revendications précédentes, **caractérisé en ce qu'**il ne comprend pas une étape de traitement du sérum approprié pour séparer le ligand de myostatine à partir du prodomaine de myostatine.

7. Procédé selon l'une quelconque des revendications précédentes, **caractérisé en ce que** le prodomaine de myostatine possède la séquence d'acide aminé SEQ ID NO : 1 et la myostatine est le ligand de myostatine présentant la séquence d'acide aminé de la SEQ ID NO:2.

8. Procédé selon l'une quelconque des revendications précédentes, **caractérisé en ce qu'**en plus de traiter le sérum, au moins un échantillon contenant une concentration connue du prodomaine de myostatine est traitée séparément par les mêmes étapes.

9. Procédé selon l'une quelconque des revendications précédentes, **caractérisé en ce qu'**il congèle le sérum avant l'analyse ou **en ce qu'**il stocke le sérum au maximum à 20°C pendant jusqu'à 48 heures.

10. Utilisation d'un kit de pièces dans un procédé selon l'une quelconque des revendications précédentes, comprenant un premier anticorps polyclonal ayant une spécificité pour le prodomaine de myostatine, dans lequel le premier anticorps est prévue pour une immobilisation sur un support, et un deuxième anticorps polyclonal ayant une spécificité pour le prodomaine de myostatine, lequel deuxième anticorps est marqué avec un atome radioactif.

11. Utilisation d'un kit de pièces selon la revendication 10, **caractérisé en ce que** le premier anticorps est immobilisé sur un support.

12. Utilisation d'un kit de pièces selon l'une quelconque des revendications 10 à 11, **caractérisé en ce que** le prodomaine de myostatine présente la séquence d'acide aminé SEQ ID NO : 1 et la myostatine est le ligand de myostatine présentant la séquence d'acide aminé SEQ ID NO : 2.

13. Utilisation d'un kit de pièces selon l'une des revendications 10 à 12, **caractérisé en ce que** le premier anticorps, à ceci près qu'il est immobilisé, est identique au deuxième anticorps, qui de plus est marqué.


### SEQUENCE LISTING

<110> Medizinische Hochschule Hannover
<120> Analysis of myostatin in serum
<130> M1057EP
<160> 2
<170> BiSSAP 1.2
<210> 1
   <211> 243
   <212> PRT
   <213> Homo sapiens
<220>
   <223> myostatin prodomain
<400> 1
   Asn Glu Asn Ser Glu Gln Lys Glu Asn Val Glu Lys Glu Gly Leu Cys
   1               5                   10                  15
   Asn Ala Cys Thr Trp Arg Gln Asn Thr Lys Ser Ser Arg Ile Glu Ala
               20                  25                  30
   Ile Lys Ile Gln Ile Leu Ser Lys Leu Arg Leu Glu Thr Ala Pro Asn
           35                  40                  45
   Ile Ser Lys Asp Val Ile Arg Gln Leu Leu Pro Lys Ala Pro Pro Leu
       50                  55                  60
   Arg Glu Leu Ile Asp Gln Tyr Asp Val Gln Arg Asp Asp Ser Ser Asp
   65                  70                  75                  80
   Gly Ser Leu Glu Asp Asp Asp Tyr His Ala Thr Thr Glu Thr Ile Ile
                   85                  90                  95
   Thr Met Pro Thr Glu Ser Asp Phe Leu Met Gln Val Asp Gly Lys Pro
               100                 105                 110
   Lys Cys Cys Phe Phe Lys Phe Ser Ser Lys Ile Gln Tyr Asn Lys Val
           115                 120                 125
   Val Lys Ala Gln Leu Trp Ile Tyr Leu Arg Pro Val Glu Thr Pro Thr
       130                 135                 140
   Thr Val Phe Val Gln Ile Leu Arg Leu Ile Lys Pro Met Lys Asp Gly
   145                 150                 155                 160
   Thr Arg Tyr Thr Gly Ile Arg Ser Leu Lys Leu Asp Met Asn Pro Gly
                   165                 170                 175
   Thr Gly Ile Trp Gln Ser Ile Asp Val Lys Thr Val Leu Gln Asn Trp
               180                 185                 190
   Leu Lys Gln Pro Glu Ser Asn Leu Gly Ile Glu Ile Lys Ala Leu Asp
           195                 200                 205
   Glu Asn Gly His Asp Leu Ala Val Thr Phe Pro Gly Pro Gly Glu Asp
       210                 215                 220
   Gly Leu Asn Pro Phe Leu Glu Val Lys Val Thr Asp Thr Pro Lys Arg
   225                 230                 235                 240
   Ser Arg Arg
<210> 2
   <211> 109
   <212> PRT
   <213> Homo sapiens
<220>
   <223> myostatin ligand
<400> 2
   Asp Phe Gly Leu Asp Cys Asp Glu His Ser Thr Glu Ser Arg Cys Cys
   1               5                   10                  15
   Arg Tyr Pro Leu Thr Val Asp Phe Glu Ala Phe Gly Trp Asp Trp Ile
               20                  25                  30
   Ile Ala Pro Lys Arg Tyr Lys Ala Asn Tyr Cys Ser Gly Glu Cys Glu
           35                  40                  45
   Phe Val Phe Leu Gln Lys Tyr Pro His Thr His Leu Val His Gln Ala
       50                  55                  60
   Asn Pro Arg Gly Ser Ala Gly Pro Cys Cys Thr Pro Thr Lys Met Ser
   65                  70                  75                  80
   Pro Ile Asn Met Leu Tyr Phe Asn Gly Lys Glu Gln Ile Ile Tyr Gly
                   85                  90                  95
   Lys Ile Pro Ala Met Val Val Asp Arg Cys Gly Cys Ser
               100                 105